# EUROPÄISCHE PATENTSCHRIFT

(11) **EP 0 441 468 B1**
(45) Veröffentlichungstag und Bekanntmachung des Hinweises auf die Patenterteilung: **11.09.1996**
(21) Anmeldenummer: 91250038.6
(22) Anmeldetag: 11.02.1991
(51) Int. Cl.: A61K 49/00, A61B 8/00, A61K 9/50

(54) **Aus Polyaldehyden aufgebaute gasenthaltende Mikropartikel als Kontrastmittel**
Gas-containing microparticles composed of polyaldehydes as contrast agents
Microparticules contenant du gaz composées de polyaldéhydes comme agent de contraste

(30) Priorität: 09.02.1990 DE 4004430
(43) Veröffentlichungstag der Anmeldung: 14.08.1991
(73) Patentinhaber: SCHERING AKTIENGESELLSCHAFT, D-13342 Berlin (DE)
(72) Erfinder: Rössling, Georg, Dr., W-1000 Berlin 28 (DE); Albayrak, Celal, Dr., W-1000 Berlin 30 (DE); Rothe, Matthias, Dr., W-1000 Berlin 42 (DE); Siegert, Joachim, Dr., W-1000 Berlin 41 (DE)

(56) Entgegenhaltungen:
- EP-A- 0 087 786
- WO-A-89/06978
- DE-A- 3 224 484
- GB-A- 2 017 125
- US-A- 4 413 070
- US-A- 4 438 239
- US-A- 4 677 138

## Beschreibung

Die Erfindung betrifft den in den Patentansprüchen gekennzeichneten Gegenstand, das heißt neue Mikropartikel, diese enthaltende pharmazeutische Mittel, deren Verwendung in der Ultraschalldiagnostik sowie Verfahren zur Herstellung dieser Mikropartikel und pharmazeutischen Mittel.

Es ist bekannt, daß durch periphere Injektion von Lösungen, die feine Gasblasen enthalten, cardiale Echokontraste erzielt werden können (Roelandt J., Ultrasound Med. Biol. 8: 471-492, 1982). Diese Gasblasen werden in physiologisch verträglichen Lösungen z.B. durch Schütteln, andere Agitation oder durch Zusatz von Kohlendioxid erhalten. Sie sind jedoch hinsichtlich Anzahl und Größe nicht einheitlich und können nur unzulänglich reproduziert werden. Auch sind sie in der Regel nicht stabilisiert, so daß ihre Lebensdauer gering ist. Ihre mittleren Durchmesser liegen meist über Erythrocytengröße, so daß keine Lungenkapillarpassage mit nachfolgender Konstrastierung von Organen wie linkes Herz, Leber, Niere oder Milz möglich ist. Darüber hinaus eignen sie sich nicht für Quantifizierungen, da sich das von ihnen erzeugte Ultraschallecho aus mehreren, nicht voneinander zu trennenden Prozessen wie Blasenentstehung, Koaleszenz und Auflösung zusammensetzt. So ist es z.B. nicht möglich, mit Hilfe dieser Ultraschall-Kontrastmittel über die Messung des Kontrastverlaufs im Myokard Aussagen über die Transitzeiten zu gewinnen.

In der EP 0 131 540 ist die Stabilisierung der Gasblasen durch Zucker beschrieben. Damit wird zwar die Reproduzierbarkeit und Homogenität des Kontrasteffektes verbessert, eine Lungenpassage überstehen diese Blasen jedoch nicht.

In den EP 0 122 624 und 0 123 235 wird beschrieben, daß der gasblasenstabilisierende Effekt von Zuckern, Zuckeralkoholen und Salzen durch Zusatz von Tensiden verbessert wird. Eine Lungenkapillargängigkeit und die Möglichkeit zur Darstellung des arteriellen Gefäßschenkels und verschiedener Organe wie Leber oder Milz ist bei diesen Ultraschallkontrastmitteln gegeben. Der Kontrasteffekt ist hierbei jedoch auf das Gefäßlumen beschränkt, da die Bläschen nicht von den Gewebezellen aufgenommen werden.

Keines der bisher bekannten Ultraschall-Kontrastmittel verbleibt längere Zeit unverändert im Körper. Eine Organdarstellung mit ausreichender Signalintensität durch selektive Anreicherung nach i.v. Gabe oder Quantifizierungen sind daher nicht möglich.

Eine Verkapselung von Gasen, wie beispielsweise Luft als Ultraschall-Kontrastmittel, wird in der DE-OS 38 03 972 beschrieben. Das hierbei verwendete Wandmaterial besteht aus bioabbaubarem synthetischem Material, wie vor allem Cyanacrylat und Polylactid.

Diese Mikropartikel sind jedoch - insbesondere in größerem Maßstab sowie im Hinblick auf ihre Aufarbeitung - schwierig herstellbar. So ist vor allem die breite Größenverteilung der Partikel von Nachteil.

Mikropartikel auf der Basis polymerisierter Aldehyde werden im US 4,413,070, US 4,438,239, DE 32 24 484, EP 0 087 786, GB 2 017 125 und im US-Patent 4,677,138 beschrieben. Die in den genannten Schriften offenbarten Partikel finden unterschiedliche Verwendung u.a. bei der Separierung von Proteinen, in der Hyperthermie und in Depotformulierungen. Allen Schriften ist gemeinsam, daß die darin offenbarten Partikel für eine Anwendung in der Ultraschalldiagnostik ungeeignet sind, da sie keine gasförmige Komponente enthalten.

Es bestand daher die Aufgabe, Ultraschall-Kontrastmittel zu schaffen, die diese Nachteile nicht aufweisen. Diese Aufgabe wird durch die vorliegende Erfindung, d.h. durch die Bereitstellung der erfindungsgemäßen Mikropartikel gelöst.

Die erfindungsgemäßen Mikropartikel mit einem Partikeldurchmesser von 0,1 bis 40 µm bestehen aus
(a) einem bioabbaubaren Polymeren, erhältlich aus einem polymerisierbaren Aldehyd und
(b) einem Gas und/oder einer Flüssigkeit mit einem Siedepunkt unter 60 °C und werden durch Trennung der gasenthaltenden von den nicht-gasenthaltenden Partikeln erhalten.

Das bioabbaubare Polymer kann gewünschtenfalls ein Copolymer sein, das aus einem polymerisierbaren Aldehyd und einem zur Copolymerisation fähigen Zusatz aufgebaut ist und/oder über Crosslinker vernetzt ist.

Gewünschtenfalls kann an das bioabbaubare Polymer, gegebenenfalls über ein Kopplungsagenz, ein Bio- oder Makromolekül gebunden sein.

Alle erfindungsgemäßen Polymeren (Homopolyaldehyde oder Copolyaldehyde) haben im allgemeinen Moleculargewichte von 1.000 - 30.000 d, bevorzugt 1.000 - 12.000 d. Die Partikel sind abbaubar in Blut und Plasma.

Die am Aufbau der Mikropartikel hauptsächlich beteiligten polymerisierten Aldehyde werden aus folgenden polymerisierbaren Aldehyden ausgewählt:
I. α,β-ungesättigten Aldehyden, wie zum Beispiel
   Acrolein
   Crotonaldehyd
   Propinaldehyd
II. α-substituierten Acroleinderivaten, wie zum Beispiel
   α-Methylacrolein
   α-Chloroacrolein
   α-Phenylacrolein
   α-Ethylacrolein
   α-Isopropylacrolein
   α-n-Butylacrolein
   α-n-Propylacrolein
III. Dialdehyden, wie zum Beispiel
   Glutaraldehyd, Succinaldehyd oder deren Derivate oder deren Mischungen mit zur Copolymerisation fähigen Zusätzen, wie zum Beispiel:
   α-substituierten Acroleinen
   β-substituierten Acroleinen
   Ethylcyanacrylaten
   Methylcyanacrylaten
   Butylcyanacrylaten
   Hexylcyanacrylaten
   Methylmetacrylaten
   Vinylalkoholen
   Acrylsäuren
   Methacrylsäuren
   Acrylsäurechloriden
   Methacrylsäurechloriden
   Acrylnitril
   Methacrylnitrilen
   Acrylamiden
   Substituierten Acrylamiden
   Hydroxymethylmethacrylaten
   Mesityloxid
   Dimethylaminoethylmethacrylaten-2-Vinylpyridinen
   N-vinyl-2-Pyrrolidinon

Bevorzugt sind dabei Acrolein und Glutaraldehyd.

Die gegebenenfalls am Aufbau der Mikropartikel beteiligten Tenside werden aus ionogenen oder nicht-ionogenen oberflächenaktiven Substanzen (Tensiden), wie z.B.
Polyethylenoxid
Polyoxyethylenpolyoxypropylenen wie
Pluronic^{(R)} F 68, Pluronic^{(R)} F 108, Pluronic^{(R)} F 127,
Polyethylenglykol, Poloxamin 908, Polaxamer 407
Carbonsäuresalzen, wie zum Beispiel: Natriumoleat
Polyoxyethylenfettsäureestern, wie zum Beispiel:
Polyoxyethylenstearat
Natriumdioctylsulfosuccinat
Polyglutaraldehydnatriumhydrogensulfit-Addukt
Polyacrolein-Natriumhydrogensulfit-Addukt
Polyvinylsulfonsäure
ausgewählt. Sie können allein oder in Form ihrer Gemische verwendet werden.

Bevorzugt sind hiervon: Polyglutaraldehydnatriumsulfit-Addukt, Polyacrolein-Natriumhydrogensulfit-Addukt, Pluronic^{(R)} F 68, Pluronic^{(R)} F 108 und Pluronic^{(R)} F 127.

Besitzen die zum Aufbau der Mikropartikel benutzten polymerisierbaren Aldehyde oberflächenaktive Eigenschaften, so kann auf die Verwendung von Tensiden verzichtet werden. Als Beispiel derartiger Aldehyde sei der Glutaraldehyd genannt.

Als in den Mikropartikeln in freier oder gebundener Form enthaltene Gase bzw. leichtflüchtige Flüssigkeiten - bevorzugt sind hierbei Flüssigkeiten mit einem Siedepunkt unter 60 °C - sind u.a. geeignet:
Ammoniak
Luft
Edelgase (Helium, Neon, Argon, Xenon, Krypton)
Schwefelhalogenide, wie zum Beispiel: Schwefelhexafluorid,
Stickstoff
Kohlenstoffoxide
Sauerstoff
Wasserstoff,
Kohlenwasserstoffe oder deren Gemische, wie zum Beispiel:
Methan
Ethan
Propan
Butan
Pentan
Neopentan
Isopentan
Cyclopentan
Ethylen
Propylen
Acetylen
3,3-Dimethyl-1-Butin
2,3-Pentadien
2-Methyl-2-Buten
2-Methyl-1,3-Butadien
2-Butin
2-Methyl-1-Buten
3-Methyl-1-Buten,
halogenierte Kohlenwasserstoffe oder Gemische, wie zum Beispiel:
Methylenchlorid
1,1-Dichlorethylen
Isopropylchlorid
Dibromdifluormethan
Brommethan,
Ether, wie zum Beispiel: Dimethylether, Diethylether oder fluorierte Ether,
oder Verbindungen wie zum Beispiel:
Dimethylaminoaceton
Propylenoxid
N-Ethylmethylamin
N-Ethyldimethylamin
Furan.

Bevorzugt sind hiervon: Luft, Argon, Xenon, Schwefelhexafluorid, Propan, Butan und Furan.

Als am Aufbau der Mikropartikel beteiligten Kopplungsagenzien sind vor allem geeignet:
I. Aminogruppenhaltige Verbindungen, wie zum Beispiel:
   Hydroxylamin
   Butylamin
   Allylamin
   Ethanolamin
   Trishydroxymethylaminomethan
   3-Amino-1-propansulfonsäure
   5-Aminovaleriansäure
   8-Aminooctansäure
   D-Glucosaminhydrochlorid
   Aminogalactose
   Aminosorbit
   Aminomannit
   Diethylaminoethylamin
   Aniline
   Sulfonilsäureamid
   Cholin
   N-Methylglucamin
   Piperazin
   1,6-Hexandiamin
   Harnstoff
   Hydrazin
   Glycin
   Alanin
   Lysin
   Serin
   Valin
   Leucin
   Peptide
   Proteine
   Albumin
   Humanserumalbumin
   Polylysin
   Gelatine
   Polyglykolamine
   Aminopolyalkohole
   Dextransulfate mit Aminogruppen
   N-Aminopolyethylenglycol (HO-PEG-NH₂)
   N,N'-Diaminopolyethylenglycol (NH₂-PEG-NH₂)
   Antikörper
   Immunoglobuline
II. Säuregruppenhaltige Verbindungen, wie z.B.: 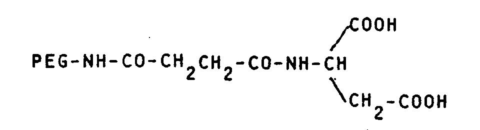 (PEG-Linker-Asparaginsäure)
   PEG-Linker-Glutaminsäure
   PEG-Linker-DTPA
   PEG-Linker-EDTA
III. Hydroxygruppenhaltige Verbindungen, wie z.B.:
   Ethylenglykol
   1,3-Propandiol
   Glycerin
   Pentaerythrit

Bevorzugt sind hiervon: Hydroxylamin, Trishydroxymethylaminomethan, 3-Amino-1-propansulfonsäure, D-Glucosaminhydrochlorid, Aminomannit, Harnstoff, Humanalbumin, Hydrazin, Proteine, Polyglykolamine, Aminopolyalkohole wie z.B. HO-PEG-NH₂ oder NH₂-PEG-NH₂ oder säuregruppenhaltige Verbindungen wie z.B. PEG-Linker-Asparaginsäure, PEG-Linker-Glutaminsäure, PEG-Linker-DTPA und PEG-Linker-EDTA, wobei das Molekular-Gewicht des Polyethylenglycols (PEG) bis 100.000 d, vorzugsweise unter 40.000 d, liegt.

Die unter I. genannten Kopplungsagenzien sind über ihre Aminogruppe an die sich auf der Oberfläche der aus polymerisierten Aldehyden und gegebenenfalls Tensiden aufgebauten Mikropartikel befindlichen Formylgruppen kondensiert.

Ebenfalls über die Formylgruppen gebunden sind die unter IV. aufgeführten Monomere, die mit weiteren Monomeren polymerisiert sein können.

Die unter II. und III. genannten Säuren und Alkohole sind dagegen erst nach vorheriger Umwandlung der Aldehydfunktion an die Mikropartikel angekoppelt.

Durch die Wahl geeigneter, über diese Kopplungsagenzien gebundenen Bio- oder Makromoleküle, wie z.B. Enzyme, Dextrane, Immunoglobuline, monoklonale Antikörper (s. weiter unten) erhält man erfindungsgemäße Mikropartikel, die eine überraschend hohe Gewebe- und Organspezifität aufweisen.

Die erfindungsgemäßen Mikropartikel weisen die eingangs geschilderten gewünschten Eigenschaften auf. Sie sind einfach und mit hoher Ausbeute herstellbar. Eine Maßstabsvergrößerung der Herstellung (up-scaling) ist ebenso wie die Reinigung der Mikropartikel problemlos.

Überraschenderweise weisen die erfindungsgemäßen Mikropartikel eine bessere Verträglichkeit auf, vor allem im Hinblick auf Herz-Kreislauf Effekte (Blutdruck, Herz-Arhythmien usw.) und zeigen kein trombogenes Potential.

Die Partikel weisen eine schmale Größenverteilung (monodispers) auf; es ist dabei möglich, die Größe der Partikel je nach eingesetzter Konzentration der Ausgangsstoffe über einen großen Bereich zu variieren (s. weiter unten). Durch Steuerung der Herstellungsbedingungen (z.B. pH-Wert) ist es möglich, auch das Molekulargewicht in großen Bereichen zu variieren.

Ein weiterer Vorteil besteht darin, daß die Reaktion zur Synthese der Mikropartikel durch viele Möglichkeiten ausgelöst werden kann, beispielsweise: Anionische Polymerisation durch pH-Änderung, kationische Polymerisation mit z.B. Eisensalzen, radikalische Polymerisation mit UV-Licht und durch ionisierende Strahlung.

Der für die Herstellung der Mikropartikel mögliche weite Temperaturbereich (-5 bis +80 °C) gestattet eine einfache Versuchssteuerung mit optimalen Ausbeuten bei sehr unterschiedlichen Gasen bzw. leichtsiedenden Flüssigkeiten.

Die Partikel enthalten freie Aldehydgruppen, die durch chemische Reaktionen mit anderen Molekülen kovalent verknüpft werden können. Diese Möglichkeit gestattet es, die Eigenschaften der Partikeloberfläche gezielt zu verändern, ohne die echogenen Eigenschaften zu beeinflussen. Durch Auswahl geeigneter Kopplungsagenzien läßt sich die kolloidale Stabilität beeinflussen. Insbesondere wird dadurch das für kolloidale Systeme häufig auftretende Phänomen der Agglomeration verhindert. Dies wiederum ist von großem Vorteil für die Entwicklung einer stabilen Formulierung.

Neben der Beeinflussung der Stabilität bieten sich Möglichkeiten, die Oberfläche der Partikel so zu verändern, daß ein drug-targeting möglich ist. Dies geschieht durch Ankoppelung geeigneter Bio- oder Makromoleküle (z.B. monoklonaler Antikörper), die eine hohe Gewebe- und Organspezifität bewirken (G. Gregoriadis, G. Poste "Targeting of Drugs", Plenum Press 1988, New York) oder durch Beeinflussung der Oberflächeneigenschaften der Partikel durch Adsorption von Molekülen (z.B. Tensiden).

In Abhängigkeit von der Wahl dieser Moleküle und von der Größe der Mikropartikel läßt sich eine Partikelanreicherung in/an Tumoren bzw. z.B. in der Lunge, Leber, Milz und im Knochenmark erreichen. Die Anreicherung im Knochenmark wird insbesondere dadurch erreicht, daß kleine Partikel (< 100 nm) mit z.B. Poloxamer 407 gecoatet werden. Sind die Partikel z.B. mit Poloxamin 908 gecoated, wird das RES-System von diesen Partikeln überwunden, und sie bleiben im Blutkreislauf (blood pool agent).

Durch mit Antikörpern gekoppelte Teilchen läßt sich eine Anreicherung der Partikel in/an Tumoren erreichen.

Die Herstellung der erfindungsgemäßen Mikropartikel erfolgt dadurch, daß man eine 0 bis 40 %, vorzugsweise 0,01 bis 10 % w/v Tensid(e) und Gase oder leichtflüchtige Flüssigkeiten enthaltende wäßrige Lösung unter Rühren, bei einer Temperatur von -5° bis +80 °C, vorzugsweise 0° bis 40 °C, einem pH-Wert von 7 bis 14, vorzugsweise 9 bis 13, innerhalb von 1 Minute bis 24 Stunden, vorzugsweise 1 Stunde bis 10 Stunden, und gegebenenfalls unter Einleiten von Gas mit copolymerisierbarem/n Aldehyd(en) bis zu einer Konzentration bezogen auf die Reaktionsmischung von 0,1 bis 50 %, vorzugsweise 3 bis 20 % w/v, sowie mit copolymerisierbaren Zusätzen einer Konzentration bezogen auf die Reaktionslösung von 0 bis 20 %, vorzugsweise 1 bis 5 % w/v, mit Crosslinker(n) einer Konzentration bezogen auf die Reaktionsmischung von 0 - 5 %, vorzugsweise 0,1 bis 1 % w/v, umsetzt,
anschließend nach Reinigung - die so erhaltenen Mikropartikel mit einer wäßrigen Lösung, die - bezogen auf die Aldehydmenge - bis zu äquimolare Mengen an Kopplungsagenz sowie 0 bis 20 % , vorzugsweise 0,01 bis 10 % w/v Tensid(e) bezogen auf das Gesamtvolumen enthält, unter Rühren bis zu 3 Tagen, vorzugsweise bis zu 2 Tagen, bei Temperaturen von 0° bis 60 °C, vorzugsweise 5° bis 30 °C, bei einem pH-Wert von 3 bis 9, vorzugsweise 5 bis 8, umsetzt und - gewünschtenfalls nach Reinigung - diese gegebenenfalls an Bio- oder Makromoleküle bindet.

Die nach der ersten Reaktionsstufe erhaltenen Polymeraldehyd-Partikel haben auf der Oberfläche Aldehydgruppen. Mit diesen Aldehydgruppen lassen sich die für Aldehyde typischen Reaktionen durchführen (R.C. Schulz, Kolloidzeitschrift und Zeitschrift für Polymere, 182 (1-2), 99 (1961); Lehrbuch der organischen Chemie "Organikum", VEB Verlag der Wissenschaften, Berlin, 1984). Dadurch ist man in der Lage, auf der Partikeloberfläche Moleküle anzukoppeln, die die Oberflächeneigenschaften ändern.

Beispiele für mögliche Reaktionen der Aldehydgruppen:
- Reduktion zu Alkohol
- Oxidation zu Säuren
- Oximierung
- Iminbildung, gegebenenfalls gefolgt von Hydrierung und gegebenenfalls anschließender N-alkylierung
- Hydrazonbildung, gegebenenfalls gefolgt von Hydrierung
- Mercaptalisierung
- Acetalisierung
- Disproportionierung durch NaOH (Cannizzaro-Reaktion)
- Aldolkondensation

Die Kopplung von aminogruppenhaltigen Molekülen an die in der ersten Reaktionsstufe gebildeten Partikel erfolgt durch Umsetzung mit den Aldehydgruppen. Hierbei wählt man beispielsweise folgende experimentelle Bedingungen:

1000 mg Polyacrolein-Partikel werden in 50 ml destilliertem Wasser suspendiert. Zu dieser Partikelsuspension werden 5000 mg der umzusetzenden Substanz zugegeben und bei Raumtemperatur gerührt. Entsprechend der Reaktionsgeschwindigkeit der Umsetzung muß gerührt werden; bei langsamen Reaktionsgeschwindigkeiten bis 48 Stunden. Die Partikelsuspension wird anschließend dialysiert (Cut off 10000 d).

Enthalten die durch z.B. die oben angegebenen Reaktionen eingeführten Substituenten (gegebenenfalls intermediär geschützte) funktionelle Gruppen, so können diese nach dem Fachmann bekannten Verfahren in für die Kopplung an Bio- oder Makromoleküle geeignete reaktive Gruppen umgewandelt werden. Bevorzugte derartige Gruppen sind beispielsweise die Maleimidobenzoyl-, 3-Sulfomaleimido-benzoyl, 4-(Maleimidomethyl)-cyclohexylcarbonyl-, 4-[3-Sulfo-(maleimido-methyl)-cyclohexylcarbonyl-, 4-(p-Maleimidophenyl)-butyryl-, 3-(2-Pyridyl-dithio)propionyl-, Methacryloyl-(pentamethylen)-amido-, Bromacetyl-, Jodacetyl-, 3-Jodpropyl-, 2-Bromethyl-, 3-Mercaptopropyl-, 2-Mercaptoethyl-, Phenylenisothiocyanat, 3-Aminopropyl-, Benzylester-, Ethylester-, t-Butylester, Amino-, C₁-C₆-Alkylamino-, Aminocarbonyl-, Hydrazino-, Hydrazinocarbonyl-, Maleimido-, Methacrylamido-, Methacryloylhydrazinocarbonyl-, Maleimidamidocarbonyl-, Halogeno-, Mercapto-, Hydrazinotrimethylenhydrazinocarbonyl-, Aminodimethylenamidocarbonyl-, Bromcarbonyl-, Phenylendiazonium-, Isothiocyanat-, Semicarbazid-, Thiosemicarbazid-, Isocyanat-Gruppe.

Eine Aminogruppe kann beispielsweise nach literaturbekannten Methoden (z.B. mit Thiophosgen in einem Zweiphasensystem, S. Scharma, Synthesis 1978, 803, D.K. Johnson, J. Med. Chem. 1989, Vol. 32, 236) in eine Isothiocyanatgruppe umgewandelt werden.

Durch Umsetzung einer Aminofunktion mit einem Halogenessigsäurehalogenid kann eine α-Halogenacetamidgruppe generiert werden (JACS 1969, Vol. 90, 4508; Chem. Pharm. Bull. 29 (1), 128, 1981), die ebenso wie z.B. die Isothiocyanatgruppe zur Kopplung an Bio- und Makromoleküle geeignet ist.

Als Substituenten, der in eine für eine Bindung an ein Makro- oder Biomolekül geeignete funktionelle Gruppe überführt werden kann, sind unter anderem Hydroxy- und Nitrobenzyl-, Hydroxy- und Carboxyalkyl- sowie Thioalkylreste mit bis zu 20 Kohlenstoffatomen geeignet. Sie werden nach dem Fachmann bekannten Literaturverfahren [Chem. Pharm. Bull. 33, 674 (1985), Compendium of Org. Synthesis Vol. 1-5, Wiley and Sons, Inc., Houben-Weyl, Methoden der organischen Chemie, Band VIII, Georg Thieme Verlag, Stuttgart, J. Biochem. 92, 1413, (1982)] in die gewünschten Substituenten (zum Beispiel mit der Amino-, Hydrazino-, Hydrazinocarbonyl-, Epoxid-, Anhydrid-, Methacryloylhydrazinocarbonyl-, Maleimidamidocarbonyl-, Halogeno-, Halogenocarbonyl-, Mercapto-, Isothiocyanatgruppe als funktioneller Gruppe) umgewandelt, wobei im Falle des Nitrobenzylrestes zunächst eine katalytische Hydrierung (zum Beispiel nach P.N. Rylander, Catalytic Hydrogenation over Platinum Metals, Academic Press 1967) zum Aminobenzylderivat vorgenommen werden muß.

Beispiele für die Umwandlung von an aromatische oder aliphatische Reste gebundenen Hydroxy- oder Aminogruppen sind die in geeigneten Lösungsmitteln wie Tetrahydrofuran, Dimethoxyethan oder Dimethylsulfoxid, zweiphasigen wäßrigen Systemen, wie z.B. Wasser/Dichlormethan, in Gegenwart eines Säurefängers wie zum Beispiel Natriumhydroxid, Natriumhydrid oder Alkali oder Erdalkalicarbonaten wie zum Beispiel Natrium-, Magnesium-, Kalium-, Calciumcarbonat oder Poly-(4-vinylpyridin) Reillex^{(R)} bei Temperaturen zwischen 0 °C und dem Siedepunkt des jeweiligen Lösungsmittels, vorzugsweise jedoch zwischen 20 °C und 60 °C, durchgeführten Umsetzungen mit einem Substrat der allgemeinen Formel I

Nf-L-Fu (I),

worin Nf für ein Nucleofug wie z.B. Cl, Br, J. CH₃C₆H₄SO₃ oder CF₃SO₃, L für einen aliphatischen, aromatischen, arylaliphatischen, verzweigten, geradkettigen oder cyclischen Kohlenwasserstoffrest mit bis zu 20 Kohlenstoffatomen und Fu für die gewünschte funktionelle Gruppe, gegebenenfalls in geschützter Form, stehen (DE-OS 34 17 413).

Als Beispiele für Verbindungen der allgemeinen Formel I seien genannt
Br(CH₂)₂NH₂, Br(CH₂)₃OH, BrCH₂COOCH₃, BrCH₂CO₂^{t}Bu, ClCH₂CONHNH₂, Br(CH₂)₄CO₂C₂H₅, BrCH₂COBr, BrCH₂CONH₂, ClCH₂COOC₂H₅, BrCH₂CONHNH₂, 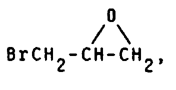 CF₃SO₃(CH₂)₃Br, BrCH₂C≡CH, BrCH₂CH=CH₂, BrCH₂C₆H₄NCS.

Umwandlungen von Carboxy-Gruppen können zum Beispiel nach der Carbodiimid-Methode (Fieser, Reagents for Organic Syntheses 10, 142), über ein gemischtes Anhydrid [Org. Prep. Proc. Int. 7, 215 (1975)] oder über einen aktivierten Ester (Adv. Org. Chem. Part B, 472) durchgeführt werden.

Die so erhaltenen kopplungsagenz-tragenden Mikropartikel können auch an Bio- oder Makromoleküle geknüpft sein, von denen bekannt ist, daß sie sich in dem zu untersuchenden Organ oder Organteil besonders anreichern. Solche Moleküle sind beispielsweise Enzyme, Hormone, Polysaccharide wie Dextrane oder Stärken, Porphyrine, Bleomycine, Insulin, Prostaglandine, Steroidhormone, Aminozucker, Aminosäuren, Peptide wie Polylysin, Proteine (wie zum Beispiel Immunoglobuline, monoklonale Antikörper, Lektine), Lipide (auch in Form von Liposomen) und Nukleotide vom DNA- oder RNA-Typ. Besonders hervorzuheben sind Konjugate mit Albuminen, wie Humanserumalbumin, Antikörpern, wie zum Beispiel monoklonale, für tumorassoziierte Antigene spezifische Antikörper oder Antimyosin. Anstelle von biologischen Makromolekülen können auch geeignete synthetische Polymere wie Polyethylenimine, Polyamide, Polyharnstoffe, Polyether wie Polyethylenglykole und Polythioharnstoffe angeknüpft werden. Die hieraus gebildeten pharmazeutischen Mittel eignen sich beispielsweise zur Anwendung in der Tumor- und Infarkt-Diagnostik sowie Tumortherapie. Monoklonale Antikörper (zum Beispiel Nature 256, 495, 1975) haben gegenüber polyklonalen Antikörpern die Vorzüge, daß sie spezifisch für eine antigene Determinante sind, eine definierte Bindungsaffinität besitzen, homogen sind (damit wird ihre Reindarstellung wesentlich einfacher) und in Zellkulturen in großen Mengen herstellbar sind. Als solche sind zum Beispiel für die Tumordarstellung monoklonale Antikörper bzw. deren Fragmente Fab und F(ab')₂ geeignet, die zum Beispiel spezifisch sind für humane Tumore des Gastrointestinaltraktes, der Brust, der Leber, der Blase, der Keimdrüsen und von Melanomen [Cancer Treatment Repts. 68, 317, (1984), Bio Sci 34, 150, (1984)] oder gegen Carcinomembryonales Antigen (CEA), Humanes Choriogonadotropin (β-HCG) oder andere tumorständige Antigene, wie Glycoproteine, gerichtet sind [New Engl. J. Med. 298, 1384, (1973), US-P 4,331,647]. Geeignet sind unter anderem auch Anti-Myosin, Anti-Insulin- und Anti-Fibrin-Antikörper (US-P 4,036,945).

Coloncarcinome lassen sich mit Hilfe von Mikropartikel-Konjugaten mit dem Antikörper 17-1A (Centocor, USA) diagnostisch nachweisen.

Im Falle der Antikörper-Konjugate darf die Bindung des Antikörpers an die Mikropartikel nicht zum Verlust oder zur Verminderung der Bindungsaffinität und Bindungsspezifität des Antikörpers zum Antigen führen. Dies kann entweder durch Bindung an den Kohlenhydrat-Anteil im Fc-Teil des Glycoproteins bzw. in den Fab oder F(ab')₂-Fragmenten oder durch Bindung an Schwefelatome des Antikörpers bzw. der Antikörper-Fragmente erfolgen.

Im ersten Fall muß zunächst eine oxidative Spaltung von Zuckereinheiten zur Generation kopplungsfähiger Formylgruppen durchgeführt werden. Diese Oxidation kann auf chemischem Wege mit Oxidationsmitteln wie z.B. Perjodsäure, Natriummetaperjodat oder Kaliummetaperjodat nach literaturbekannten Methoden (zum Beispiel J. Histochem and Cytochem. 22, 1084, 1974) in wäßriger Lösung in Konzentrationen von 1 bis 100, vorzugsweise 1 bis 20 mg/ml, und einer Konzentration des Oxidationsmittels zwischen 0,001 bis 10 mMol, vorzugsweise 1 bis 10 mMol, in einem pH-Bereich von ca. 4 bis 8 bei einer Temperatur zwischen 0 bis 37 °C und einer Reaktionsdauer zwischen 15 Minuten und 24 Stunden vorgenommen werden. Auch auf enzymatischem Wege kann die Oxydation, beispielsweise mit Hilfe von Galaktoseoxidase, in einer Enzymkonzentration von 10 - 100 Einheiten/ml, einer Substratkonzentration von 1 bis 20 mg/ml, bei einem pH-Wert von 5 bis 8, einer Reaktionsdauer von 1 bis 8 Stunden und einer Temperatur zwischen 20 und 40 °C, durchgeführt werden (zum Beispiel J. Biol. Chem. 234, 445, 1959).

An die durch Oxidation generierten Aldehyde werden Mikropartikel mit geeigneten funktionellen Gruppen, wie zum Beispiel Hydrazin, Hydrazid, Hydroxylamin, Phenylhydrazin, Semicarbazid und Thiosemicarbazid, durch Reaktion zwischen 0 - 37 °C, bei einer Reaktionsdauer von 1 bis 65 Stunden, einem pH-Wert zwischen ca. 5,5 und 8, einer Antikörperkonzentration von 0,5 bis 20 mg/ml und einem molaren Verhältnis des Komplexbildners zum Antikörperaldehyden von 1:1 bis 1000:1 gebunden. Die anschließende Stabilisierung des Konjugats erfolgt durch Reduktion der Doppelbindung, z.B. mit Natriumborhydrid oder Natriumcyanoborhydrid; das Reduktionsmittel wird dabei in einem 10 bis 100fachen Überschuß verwendet (zum Beispiel J. Biol. Chem. 254, 4359, 1979).

Die zweite Möglichkeit der Bildung von Antikörper-Konjugaten geht aus von einer schonenden Reduktion der Disulfid-Brücken des Immunoglobulin-Moleküls; hierbei werden die empfindlicheren Disulfid-Brücken zwischen den H-Ketten des Antikörper-Moleküls gespalten, während die S-S-Bindungen der Antigenbindenden Region intakt bleiben, so daß praktisch keine Verminderung der Bindungsaffinität und -spezifität des Antikörpers eintritt (Biochem. 18, 2226, 1979, Handbook of Experimental Immunology, Vol. 1, Second Edition, Blackwell Scientific Publications, London 1973, Chapter 10). Diese freien Sulfhydryl-Gruppen der interHKetten Regionen werden dann mit geeigneten funktionellen Gruppen der Mikropartikel bei 0 bis 37 °C, einem pH-Wert von ca. 4 bis 7, und einer Reaktionsdauer von 3 bis 72 Stunden unter Ausbildung einer kovalenten Bindung, die die Antigen-Bindungsregion des Antikörpers nicht beeinflußt, umgesetzt. Als geeignete reaktive Gruppen seien beispielsweise genannt: Halogenalkyl-, Halogenacetyl-, p-Mercuribenzoat-, Isothiocyanat-, Thiol-, Epoxidgruppen sowie Gruppen, die einer Michael-Additions-Reaktion, wie zum Beispiel Maleinimide, Methacrylogruppen (zum Beispiel J. Amer. Chem. Soc. 101, 3097, 1979), zu unterwerfen sind.

Zur Verknüpfung der Antikörperfragmente mit den Mikropartikeln gibt es zusätzlich eine Reihe geeigneter, oft auch kommerziell erhältlicher bifunktioneller "Linker" (siehe zum Beispiel Pierce, Handbook and General Catalogue 1986), die sowohl gegenüber den SH-Gruppen der Fragmente als auch gegenüber den Amino- bzw. Hydrazinogruppen der Mikropartikel reaktiv sind.

Als Beispiele seien genannt:
m-Maleimidobenzoyl-N-hydroxysuccinimidester (MBS),
m-Maleimidobenzoyl-N-sulfosuccinimidester (Sulfo-MBS),
N-Succinimidyl-[4-(Iodacetyl)-amino]benzoesäureester (SIAB),
Succinimidyl-4(N-maleimidomethyl)-cyclohexan-1-carbonsäureester (SMCC),
Succinimidyl-4(p-maleimidophenyl)-buttersäureester (SMPB),
N-Succinimidyl-3-(2-pyridyldithio)-propionsäureester (SDPD),
4-[3-(2,5-Dioxo-3-pyrrolinyl)-propionyloxy]-3-oxo-2,5-diphenyl-2,3-dihydrothiophen-1,1-dioxid,
Acetylalanylleucylalanylaminopbenzyl,
Acetamido-p-thioureidobenzyl.

Es können auch Bindungen nicht-kovalenter Art zur Kopplung an das Bio-oder Makromolekül genutzt werden, wobei sowohl ionische als auch van der Waals-und Wasserstoffbrücken-Bindungen in wechselnden Anteilen und Stärke (Schlüssel-Schloß-Prinzip) zur Bindung beitragen können (zum Beispiel Avidin-Biotin, Antikörper-Antigen). Auch Einschlußverbindungen (host-guest) kleinerer Komplexe in größere Cavitäten beim Makromolekül sind möglich.

Das Kopplungsprinzip besteht darin, zunächst ein bifunktionelles Makromolekül herzustellen, indem man entweder ein gegen ein Tumorantigen gerichtetes Antikörper-Hybridom mit einem gegen die erfindungsgemäßen Mikropartikel gerichtetes zweiten Antikörper-Hybridom fusioniert oder die beiden Antikörper chemisch über einen Linker (beispielsweise in der im J. Amer. Chem. Soc. 101, 3097 (1979) angegebenen Weise) miteinander verknüpft oder den gegen das Tumorantigen gerichteten Antikörper, gegebenenfalls über einen Linker, an Avidin (bzw. Biotin) bindet [D.J. Hnatowich et al., J. Nucl. Med. 28, 1294 (1987)]. Anstelle der Antikörper können auch ihre entsprechenden F(ab)- bzw. F(ab')₂-Fragmente verwendet werden. Für die pharmazeutische Anwendung injiziert man zunächst das bifunktionelle Makromolekül, das sich am Zielort anreichert, und dann im zeitlichen Abstand die erfindungsgemäßen Mikropartikel [gegebenenfalls an Biotin (bzw. Avidin) gebunden], die in-vivo am Zielort angekoppelt werden und dort ihre diagnostische oder therapeutische Wirkung entfalten können. Darüberhinaus können auch andere Kopplungsmethoden wie beispielsweise das in Protein Tailoring Food Med. Uses [Am. Chem. Soc. Symp.] (1985), 349, beschriebene "Reversible Radiolabeling" zur Anwendung kommen.

Mit der sogenannten Festphasen-Kopplung steht eine besonders einfache Methode zur Herstellung von Antikörper-Konjugaten bzw. Antikörperfragment-Konjugaten zur Verfügung: Der Antikörper wird an eine stationäre Phase (z.B. einen Ionenaustauscher), der sich zum Beispiel in einer Glassäule befindet, gekoppelt. Durch sukzessives Spülen der Säule mit einer zur Generierung von Aldehyd-Gruppen geeigneten Lösung, Waschen, Spülen mit einer Lösung der funktionalisierten Mikropartikel, Waschen und schließlich Eluieren des Konjugats werden sehr hohe Konjugat-Ausbeuten erhalten.

Dieses Verfahren erlaubt die automatische und kontinuierliche Produktion beliebiger Mengen an Konjugaten.

Auch andere Kopplungsschritte können auf diese Art und Weise durchgeführt werden.

So können zum Beispiel durch die Sequenz Papain-Reduktion/bifunktioneller Linker/funktionalisierte Mikropartikel Fragment-Konjugate hergestellt werden.

Die so gebildeten Verbindungen werden anschließend vorzugsweise chromatographisch gereinigt.

Es lassen sich Partikel in der Größe von 0,04 - 100 µm, vorzugsweise 0,1 - 40 µm, herstellen. Die Größe der Partikel läßt sich im wesentlichen beeinflussen durch Variation der Ausgangskonzentration von Monomer, Tensid und pH-Wert.

Beispiele für die Herstellung von Partikeln bestimmter Größe:
1.)

| | |
|---|---|
| Acroleinkonzentration: | 10 % (W/V) |
| Tensidkonzentration: | 1,5 % (W/V) |
| pH-Wert: | 10,0 |
| Temperatur: | 4 °C |

Werden diese Bedingungen gewählt, so erhält man Partikel mit einem mittleren Durchmesser von 750 nm.
2.)

| | |
|---|---|
| Acroleinkonzentration: | 20 % (W/V) |
| Tensidkonzentration: | 0,2 % (W/V) |
| pH-Wert: | 10,0 |
| Temperatur: | 2 °C |

Unter diesen Bedingungen erhält man Partikel mit einem mittleren Durchmesser von 40 µm.
3.) Bei gleichen Bedingungen wie unter 2.) aufgeführt, jedoch bei einer Acroleinkonzentration von 10 % (W/V) erhält man Partikel mit einem mittleren Durchmesser von 8 µm.
4.)

| | |
|---|---|
| Acroleinkonzentration: | 10 % (W/V) |
| Tensidkonzentration: | 0,5 % (W/V) |
| pH-Wert: | 11,0 |
| Mittlerer Partikeldurchmesser: | 560 nm |

5.) Unter gleichen Bedingungen wie unter 4.), nur bei einem pH-Wert von 9 ergibt sich eine mittlere Partikelgröße von 3,2 µm.

Als Tensid wird Polyglutaraldehydnatriumhydrogensulfit-Addukt (PGL) verwendet.

Anstelle des Polyglutaraldehydnatriumhydrogensulfit-Addukts (PGL) kann auch Polyacroleinnatriumhydrogensulfit-Addukt (PAC-SO₃) eingesetzt werden, ohne daß ein Einfluß auf die Partikelgröße zu beobachten ist.

### Synthese von PGL:

Eine 25 % wäßrige Lösung von Glutaraldehyd wird über Aktivkohle gereinigt. Anschließend wird durch die Einleitung von N₂ in die wäßrige Lösung die Lösung von O₂ befreit. Ferner wird eine Pufferlösung (Phosphatpuffer, 1 molar) auf pH = 11 eingestellt. Die Pufferlösung wird auch von O₂ durch Einleitung von N₂ befreit. Die Pufferlösung und Glutaraldehydlösung werden zusammengebracht und unter N₂-Atmosphäre 72 Stunden lang polymerisiert. Danach wird das Polymerisat filtriert und mit Aceton und Wasser gewaschen. Das gewaschene Polymerisat wird im Vakuumtrockenschrank bei 45 °C getrocknet. In 30 ml H₂O, das 12,5 g NaHSO₃ enthält, werden 5 g Polyglutaraldehyd gelöst. Die Lösung wird mit destilliertem H₂O dialysiert. Anschließend wird die Lösung lyophilisiert.

Die erfindungsgemäßen Partikel lassen sich in wäßrigen Lösungen suspendieren, ohne daß es zu Aggregation der Teilchen kommt. Zur Herstellung einer galenischen Formulierung, die parenteral applizierbar ist, lassen sich wäßrige Lösungen verwenden, die isotonisierende Zusätze wie Natriumchlorid, Zuckeralkohole (Mannit, Sorbit, Xylit etc.) oder Zucker (Glucose, Fructose) enthalten. Zur Einstellung des pH-Wertes können Puffer wie Trometamol/HCl, Citronensäure/NaOH etc. gewählt werden.

### Synthese von PAC-SO₃:

A. In einem Dreihalskolben versehen mit Tropftrichter und Rührer werden 100 ml destilliertes Wasser gegeben und durch Einleiten von Stickstoff von Sauerstoff befreit. Danach werden 1,829 g K₂S₂O₈ zu diesem Wasser gegeben und gelöst. Nachdem sich K₂S₂O₈ vollständig aufgelöst hat, wird zu dieser Lösung 20 ml frisch destilliertes Acrolein gegeben. Danach wird 1,14 g AgNO₃ gelöst in 5 ml Wasser zugegeben und unter Rühren 2 Stunden lang polymerisiert. Das ausgefallene Polymerisat wird abfiltriert, mehrfach mit Wasser gewaschen und anschließend wird zur Entfernung der Silberionen 1 l Wasser, in dem 1,63 g Natriumthiosulfat gelöst sind, 1 Stunde lang resuspendiert. Das Polymerisat wird abfiltriert und im Vakuumtrockenschrank bei 45 °C getrocknet. Das getrocknete Polymerisat wird in einem Mörser grob zerkleinert. 10 g zerkleinertes Polymerisat werden in 100 ml Natriumhydrogensulfit (37 %) gelöst. Danach wird die Lösung gegen destilliertes Wasser dialysiert (cut off 5000 d). Das Dialysat wird als Tensid für die Herstellung der Polyacrolein-Mikropartikel eingesetzt.
B. In einem Rundkolben werden 100 ml destilliertes Wasser vorgelegt. Unter Rühren wird 20 ml frisch destilliertes Acrolein zugegeben. Anschließend wird durch Zugabe von NaOH-Lösung (2 N) der pH des Reaktionsgemischs auf 10,5 eingestellt und dies unter Rühren 2 Stunden lang polymerisiert. Das ausgefallene Polymerisat wird abfiltriert, mehrmals mit Wasser gewaschen und im Vakuumtrockenschrank bei 45 °C getrocknet. 10 g Polymerisat werden in 100 ml NaHSO₃-Lösung (37 %) gelöst. Die Lösung wird gegen bidestilliertes Wasser dialysiert (cut off 5000 d). Der Rückstand wird als Tensid bei der Herstellung der Polyacrolein-Mikropartikel verwendet.

### Beispiele

1.) In 100 ml einer Formulierung sind enthalten:

| | |
|---|---|
| Partikel: | 100 mg |
| Trometamol: + HCL für pH = 7,4 | 2,4 mg |
| Mannit: | 5500 mg |
| Wasser: | ad 100 ml |

2.) In 100 ml einer Formulierung sind enthalten:

| | |
|---|---|
| Partikel: | 50 mg |
| Natriumchlorid: | 860 mg |
| Wasser | ad 100 ml |

Ein Aufschwimmen der Partikel kann dadurch verhindert werden, daß die mittlere Dichte des Partikels an die des umgebenden Vehikels angeglichen wird.

Dies kann durch Zusätze von Substanzen höherer Dichte (Röntgenkontrastmittel, Magnetite) erreicht werden. Diese Möglichkeit bietet sich besonders bei Partikeln mit geringem Polyaldehydgehalt an.

Die erfindungsgemäßen pharmazeutischen Mittel enthalten 0,1 µg - 100 mg Mikropartikel/ml, vorzugsweise 10 µg - 1 mg Mikropartikel/ml galenischer Formulierung und werden in der Regel in Dosen von 0,01 ml - 10 ml/kg, vorzugsweise 0,1 - 1 ml/kg Körpergewicht, dosiert. Sie sind zur enteralen und parenteralen Applikation bestimmt.

Die nachfolgenden Beispiele sollen die Erfindung erläutern, ohne sie auf diese zu beschränken.

### Methoden zur Herstellung des Kontrastmittels

### 1. Reaktionsstufe:

A) Eine tensidhaltige (0,01 - 5 % w/v) wäßrige Lösung wird unter Rührung auf 0 °C abgekühlt. Dabei wird ein Gas in die Lösung eingeleitet. Der pH-Wert der Lösung wird mit NaOH auf den gewünschten pH-Wert (vorzugsweise 9 - 13) eingestellt. Zu dieser Lösung wird das Monomer bzw. Monomergemisch gegeben. Nach 30 Minuten wird die Rührgeschwindigkeit reduziert. Nach 1 Stunde wird das Reaktionsgemisch mit der oben angegebenen tensidhaltigen wäßrigen Lösung verdünnt. Die Rührgeschwindigkeit wird noch weiter gesenkt. Nach 4 Stunden werden die ausgefallenen nicht-gasenthaltenden Mikropartikel von der übrigen Suspension dekantiert und verworfen. Die dekantierte Suspension wird dialysiert, um das Kontrastmittel von Restmonomeren zu reinigen.
   Ausbeute: 80 - 90 %
B) Eine wäßrige Lösung, welche die gewünschte Tensid- und Monomermenge enthält, wird auf 0 °C abgekühlt. Dabei wird das gewünschte Gas unter Rühren durch die Lösung eingeleitet. Anschließend wird mit NaOH der pH-Wert der Lösung auf vorzugsweise 9 - 13 eingestellt. Nach 1 Stunde wird das Reaktionsgemisch verdünnt. Nach 3 - 4 Stunden wird die Mikropartikel enthaltende Suspension von dem ausgefallenen Polymerisat, das verworfen wird, getrennt. Die Suspension wird durch Dialyse gereinigt.
   Ausbeute: 80 - 90 %

### Beispiel 1

Es werden 91 ml 0,5 %ige wäßrige Tensidlösung in einen Kolben gegeben. Der pH-Wert der Lösung wird mit 0,2 N NaOH-Lösung auf 11 eingestellt. Durch die Lösung wird N₂ eingeleitet. Zu der auf 0 °C abgekühlten 0,5 %igen Tensidlösung wird 9,5 ml frisch destilliertes Acrolein getropft. Nach 1 Stunde werden zum Reaktionsgemisch weitere 100 ml 0,5 %ige Tensidlösung gegeben. Nach 3 Stunden wird die Mikropartikel enthaltende Suspension von den ausgefallenen Polymeren dekantiert und durch Dialyse gereinigt.

### Beispiel 2

Es werden 82 ml 0,08 %ige wäßrige Tensidlösung in einen Kolben gegeben. Die Lösung wird auf 0 °C abgekühlt. Zu der abgekühlten Lösung werden 18 ml frisch destilliertes Acrolein zugegeben. In die Lösung wird unter Rühren Argon eingeleitet. Nach 1 Stunde wird der pH-Wert der Lösung mit einer 0,2 N NaOH-Lösung auf 12 eingestellt. Nach 2 Stunden werden 100 ml 0,08 %ige Tensidlösung zugegeben. Nach 3 Stunden wird die Suspension dekantiert und dialysiert.

### Beispiel 3

Es werden 70 ml 0,08 %ige wäßrige Tensidlösung, die 10 % Dimethylformamid enthält, in einen Kolben gegeben. Der pH-Wert der Lösung wird mit 0,2 N NaOH-Lösung auf 11,5 eingestellt. Die Lösung wird auf 0 °C abgekühlt. Dabei wird N₂ in die Lösung eingeleitet. In diese Lösung werden 30 ml frisch destilliertes Acrolein getropft. Nach 1 Stunde wird der Lösung 100 ml 0,08 %ige Tensidlösung zugegeben. Nach 4 Stunden wird die Suspension von den ausgefallenen Polymeren getrennt und gereinigt.

### Beispiel 4

Es werden 91 ml 0,5 %ige wäßrige Tensidlösung, die 5 % Magnetit enthält, in einem Kolben auf 0 °C abgekühlt. Der pH-Wert der Lösung wird mit 0,2 N NaOH auf 12 eingestellt. Durch die Lösung wird N₂ eingeleitet. Zu der auf 0 °C abgekühlten Lösung werden 9 ml frisch destilliertes Acrolein zugetropft. Nach 1 Stunde werden dem Reaktionsgemisch 100 ml der 0,5 %igen Tensidlösung zugegeben. Die Mikropartikel enthaltende Suspension wird durch Dekantieren von ausgefallenen Polymeren getrennt und dialysiert.

### Beispiel 5

Es werden 91 ml 0,5 %ige Tensidlösung in einen Kolben gegeben. Der pH-Wert der Lösung wird durch Zugabe von 0,2 N NaOH-Lösung auf 12 eingestellt. Die Lösung wird auf 0 °C abgekühlt. Durch die Lösung wird Argon eingeleitet. Zu dieser Lösung werden 9 ml frisch destilliertes Acrolein, welches 5 % Butylcyanoacrylat enthält, zugetropft. Nach 1 Stunde werden weitere 100 ml der 0,5 %igen Tensidlösung zugegeben. Die Suspension wird vom Bodensatz getrennt und gereinigt.

### Beispiel 6

Es werden 91 ml 0,08 %ige wäßrige Tensidlösung in einen Kolben gegeben. Der pH-Wert der Lösung wird durch Zugabe von 0,2 N NaOH-Lösung auf 10,5 eingestellt. Die Lösung wird auf 0 °C abgekühlt. Durch die Lösung wird N₂ eingeleitet. Zu dieser Lösung werden 9 ml frisch destilliertes Acrolein, welches 20 % α-Methylacrolein enthält, zugetropft. Nach 1 Stunde werden weitere 100 ml der 0,08 %igen Tensidlösung zugegeben. Nach 2 Stunden wird die Mikrosphären-Suspension vom Bodensatz getrennt und gereinigt.

### Beispiel 7

Es werden 91 ml 0,08 %ige wäßrige Tensidlösung, welche 25 % Isopentan enthält, in einen Kolben gegeben. Die Lösung wird auf 0 °C abgekühlt. Zu dieser Lösung werden 9 ml frisch destilliertes Acrolein unter Rühren zugegeben. Nach 2 Stunden wird das Reaktionsgemisch filtriert. Die Mikropartikel werden durch Waschen mit Wasser gereinigt. Die Mikrosphären werden in Wasser resuspendiert.
Tensidlösung: Polyglutaraldehydnatriumhydrogensulfit-Addukt.

### 2. Reaktionsstufe

Eine Suspension von Polyacrolein-Mikropartikeln in destilliertem Wasser wird durch Zugabe von 0,01 N HCl-Lösung auf einen pH-Wert von 6,5 eingestellt. Zu dieser Suspension wird unter Rühren bei Raumtemperatur ein Überschuß des aminhaltigen Liganden gegeben. Der pH-Wert dieser Lösung wird zuvor durch Zugabe von 0,01 N NaOH-Lösung auf 8 eingestellt.

Im Anschluß rührt man in Abhängigkeit der Reaktionsgeschwindigkeit bis zu 48 Stunden bei Raumtemperatur. Zur Entfernung des überschüssigen aminhaltigen Liganden wird gegen Wasser dialysiert.

Gegebenenfalls werden die gebildeten Schiffschen Basen durch Zugabe von Reduktionsmitteln zu den Aminen reduziert.

### Beispiel 6

1000 mg Polyacrolein-Mikropartikel aus Beispiel 1 werden in 50 ml Wasser resuspendiert. Zu dieser Suspension werden 1000 mg 3-Aminopropan-1-sulfonsäure zugegeben und 48 Stunden bei Raumtemperatur gerührt. Danach wird die Suspension gegen Wasser dialysiert.

Dann wird mit 150 mg NaBH₃CN versetzt und bei pH 7,5 24 Stunden gerührt. Diese Suspension wird anschließend gegen Wasser dialyisert.

Gegebenenfalls kann das Amin alkyliert oder mit Chloressigsäure, Acetanhydrid oder Diglycolsäureanhydrid acetlyiert werden.

### Beispiel 9

1000 mg Polyacrolein-Mikropartikel aus Beispiel 2 werden in 50 ml Wasser resuspendiert. Zu dieser Suspension werden 1000 mg 3-Aminopropanphosphat zugegeben und 48 Stunden bei Raumtemperatur gerührt. Danach wird die Suspension gegen Wasser dialysiert.

Dann wird mit 150 mg NaBH₃CN versetzt und bei pH 7,5 24 Stunden gerührt. Diese Suspension wird anschließend gegen Wasser dialyisert.

Gegebenenfalls kann das Amin alkyliert oder mit Chloressigsäure, Acetanhydrid oder Diglycolsäureanhydrid acetlyiert werden.

### Beispiel 10

1000 mg Polyacrolein-Mikropartikel aus Beispiel 3 werden in 50 ml Wasser resuspendiert. Zu dieser Suspension werden 1000 mg 8-Aminooctansäure zugegeben und 24 Stunden bei Raumtemperatur gerührt. Danach wird die Suspension gegen Wasser dialysiert.

Dann wird mit 150 mg NaBH₃CN versetzt und bei pH 7,5 24 Stunden gerührt. Diese Suspension wird anschließend gegen Wasser dialyisert.

Gegebenenfalls kann das Amin alkyliert oder mit Chloressigsäure, Acetanhydrid oder Diglycolsäureanhydrid acetlyiert werden.

### Beispiel 11

1000 mg Polyacrolein-Mikropartikel aus Beispiel 4 werden in 50 ml Wasser resuspendiert. Zu dieser Suspension werden 1000 mg 5-Aminovaleriansäure zugegeben und 36 Stunden bei Raumtemperatur gerührt. Danach wird die Suspension gegen Wasser dialysiert.

Dann wird mit 150 mg NaBH₃CN versetzt und bei pH 7,5 24 Stunden gerührt. Diese Suspension wird anschließend gegen Wasser dialyisert.

Gegebenenfalls kann das Amin alkyliert oder mit Chloressigsäure, Acetanhydrid oder Diglycolsäureanhydrid acetlyiert werden.

### Beispiel 12

1000 mg Polyacrolein-Mikropartikel aus Beispiel 5 werden in 50 ml Wasser resuspendiert. Zu dieser Suspension werden 1000 mg D-Glucoseaminhydrochlorid zugegeben und 30 Stunden bei Raumtemperatur gerührt. Danach wird die Suspension gegen Wasser dialysiert.

Dann wird mit 150 mg NaBH₃CN versetzt und bei pH 7,5 24 Stunden gerührt. Diese Suspension wird anschließend gegen Wasser dialyisert.

Gegebenenfalls kann das Amin alkyliert oder mit Chloressigsäure, Acetanhydrid oder Diglycolsäureanhydrid acetlyiert werden.

### Beispiel 13

1000 mg Polyacrolein-Mikropartikel aus Beispiel 6 werden in 50 ml Wasser resuspendiert. Zu dieser Suspension werden 1000 mg Hexamethylendiamin zugegeben und 24 Stunden bei Raumtemperatur gerührt. Danach wird die Suspension gegen Wasser dialysiert.

Dann wird mit 150 mg NaBH₃CN versetzt und bei pH 7,5 24 Stunden gerührt. Diese Suspension wird anschließend gegen Wasser dialyisert.

Gegebenenfalls kann das Amin alkyliert oder mit Chloressigsäure, Acetanhydrid oder Diglycolsäureanhydrid acetlyiert werden.

### Beispiel 14

1000 mg Polyacrolein-Mikropartikel aus Beispiel 7 werden in 50 ml Wasser resuspendiert. Zu dieser Suspension werden 1000 mg Polylysin (MG = 32.600 Dalton) zugegeben und 30 Stunden bei Raumtemperatur gerührt. Danach wird die Suspension mit Wasser gewaschen.

### Beispiel 15

100 mg Polyacrolein-Mikropartikel aus Beispiel 5 werden in 2,5 ml Wasser resuspendiert. Zu dieser Suspension werden 250 mg in 2,5 ml Wasser gelöstes Humanserumalbumin gegeben und 8 Stunden bei Raumtemperatur gerührt. Danach wird die Suspension gegen destilliertes Wasser dialysiert (cut off 100.000).

### Beispiel 16

1000 mg Polyacrolein-Mikropartikel aus Beispiel 4 werden in 50 ml Wasser resuspendiert. Zu dieser Suspension werden 1000 mg (2-Diethylamino)-ethylamin gegeben und 20 Stunden bei Raumtemperatur gerührt. Danach wird die Suspension gegen Wasser dialysiert.

### Beispiel 17

300 mg Polyacrolein-Mikropartikel aus Beispiel 1 werden in 7,5 ml destilliertem Wasser suspendiert. Zu dieser Suspension werden 750 mg in 7,5 ml Wasser gelöste 3-Aminopropan-1-sulfonsäure gegeben und 24 Stunden bei Raumtemperatur gerührt. Danach wird die Suspension gegen destilliertes Wasser dialysiert (cut off 1000 d).

### Beispiel 18

200 mg Polyacrolein-Mikropartikel aus Beispiel 7 werden in 5 ml destilliertem Wasser resuspendiert. Zu dieser Suspension werden 500 mg in 5 ml Wasser gelöstes Lysin gegeben und 24 Stunden bei Raumtemperatur gerührt. Danach wird die Suspension gegen destilliertes Wasser dialysiert (cut off 5000 d).

### In-vitro-Versuche

In in-vitro-Experimenten werden durch Rückstreumessungen der Echoamplitude von Suspensionen beispielhaft ausgewählter erfindungsgemäßer Mikropartikel deren sehr gute akustische Eigenschaften belegt.

Zur Erklärung der in-vitro-Versuche und der daraus gewonnenen Abbildungen:

Die Meßapparatur besteht aus einem Ultraschallsender kombiniert mit einem Ultraschallempfänger und einer Meßküvette mit der Probe. Zur Messung der akustischen Eigenschaften der Probe wird ein Ultraschallimpuls ausgesendet. Der Impuls wird an der Glaswand der Küvette gestreut, durchläuft die Probe und wird dann, falls die Probe nicht echogen ist, an der Rückseite der Küvette gestreut. Die Rückstreuung des Ultraschallimpulses wird vom Empfänger gemessen und durch eine Änderung der Amplitude (siehe Abbildungen) angezeigt.

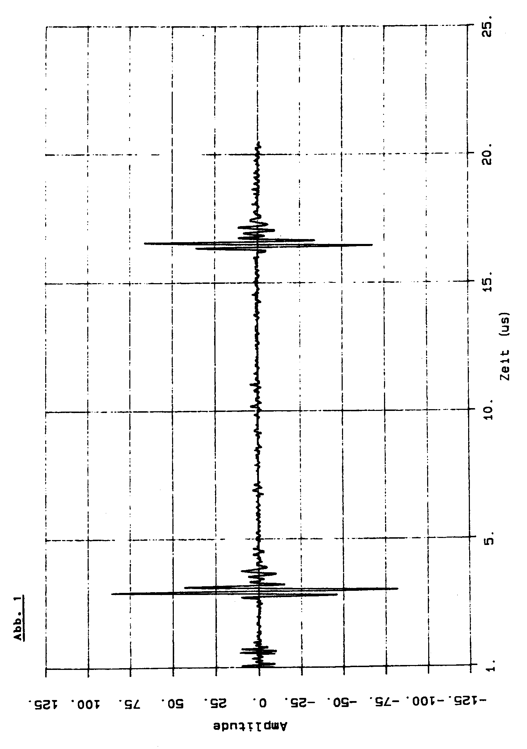

In Abb. 1 ist das Rückstreuverhalten von Wasser (als Beispiel für eine nicht echogene Probe) dargestellt. Es ist deutlich die Rückstreuamplitude der Vorderwand (bei 3 µsec) und der Rückwand (bei ca. 16 µsec) der Küvette erkennbar.

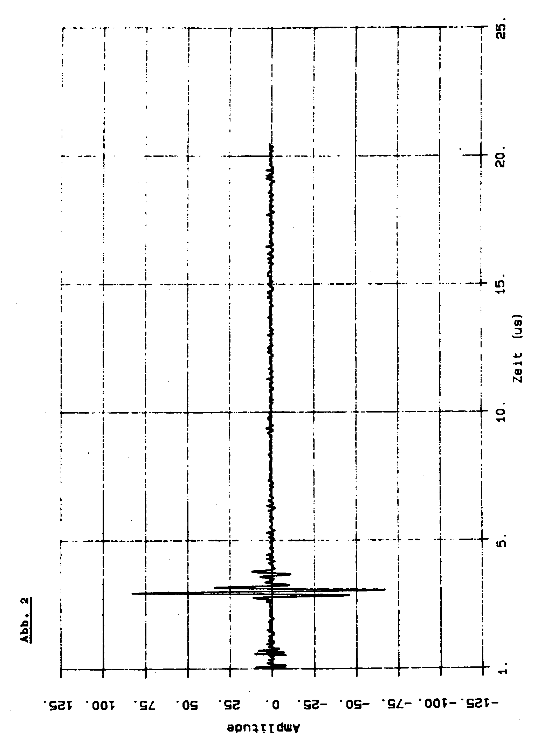

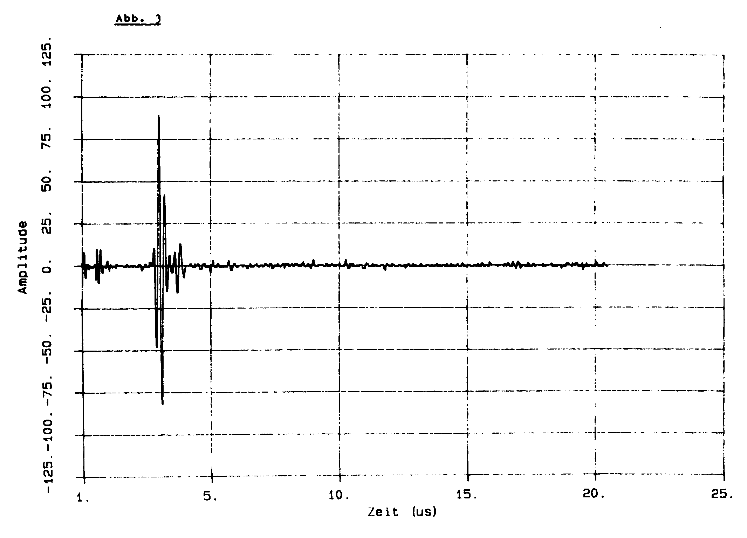

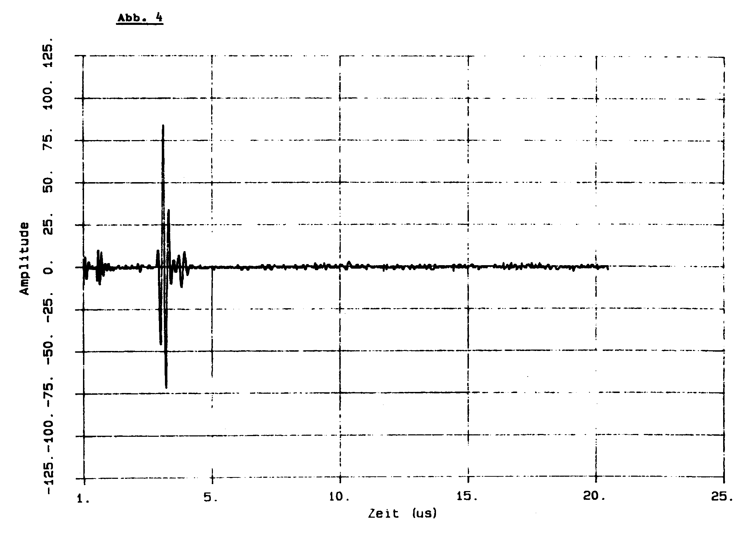

Wird eine echogene Probe vermessen, so ergibt sich ein Rückstreuverhalten wie es in den Abb. 2 - 4 wiedergegeben ist. Das Rückstreusignal der Küvettenwand wird nicht erhalten, da der Ultraschallimpuls durch Wechselwirkung mit der echogenen Probe dissipiert bzw. so verändert wird, daß keine Rückstreuung mehr zum Empfänger erfolgen kann.

Es wurden die Rückstreuamplituden von wäßrigen Partikelsuspensionen der Beispiele 8 (Abb. 2), 11 (Abb. 3) und 15 (Abb. 4), jeweils in einer Konzentration von 0,5 mg/ml, bestimmt.

### In-vivo-Versuche

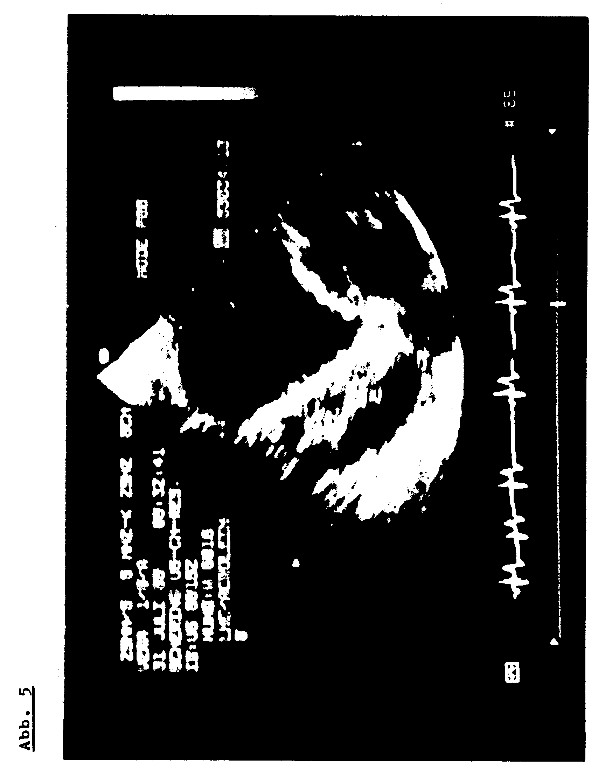

Zur Durchführung einer echokardiographischen Untersuchung bei einem ca. 10 kg schweren Hund (Beagle) werden die erfindungsgemäßen Kontrastmittel folgenderweise verwendet: Aus dem Vial mit der gebrauchsfertigen Suspension wird 1 ml der Lösung entnommen, die 40 µg/ml mit Albumin gekoppelte Partikel (Beispiel 15) in 5 % Glukoselösung enthält. Die Injektion dieses Kontrastmittels erfolgt in die Vena saphena ramus caudalis über einen allseitig offenen Dreiwegehahn mit einer Injektionsgeschwindigkeit von mindestens 1 ml/s, günstiger jedoch mit einer Geschwindigkeit von 3 ml/s, gefolgt von einer Nachinjektion von 5 ml einer physiologischen Kochsalzlösung (0,9 %ig). Die Nachinjektion erfolgt, um einen möglichst lange bestehenbleibenden Kontrastmittelbolus zu erhalten. Vor der Injektion (Abb. 5) wird ein "apikaler Vierkammerblick" bei dem Versuchstier mit einem handelsüblichen Schallkopf für die Echokardiographie an der Thoraxwand (transthorakale Ableitung) eingestellt und mit einer Klammer fixiert. Vor, während und nach der Injektion wird die Schallableitung auf dem Bildschirm des Ultraschall-Untersuchungsgerätes angezeigt und ggf. auf Videoband oder mit einem Videoprinter dokumentiert. Diese Versuchsanordnung entspricht dem Stand der Technik und ist dem Fachmann bekannt.

Bei Erreichen des Ultraschall-Kontrastmittels im rechten Herz lassen sich die Kontrasteffekte im Farbdoppler, im 2D-Echobild oder im M-Mode Echobild verfolgen. Das Kontrastmittel markiert zuerst das Blut des rechten Vorhofes, dann wird der rechte Ventrikel und schließlich die Pulmonalarterie kontrastiert. Hierbei tritt eine homogene Füllung auf, die für eine diagnostische Untersuchung ausreichende Zeit anhält. Während die Höhlen des rechten Herzens sich wieder mit nicht kontrastiertem Blut füllen (Abnahme und Verschwinden der Echogenität in den Herzhöhlen) erscheint das Kontrastmittel nach der Lungenpassage (transkapillär) in den Pulmonalvenen, füllt dann den linken Vorhof, den linken Ventrikel und das nachfolgende Hochdrucksystem homogen. Die Kontrasteffekte in den Höhlen des linken Herzens halten länger an als die auf der rechten Herzseite. Neben der Kontrastierung der Höhlen des linken Herzens erfolgt auch eine Kontrastierung anderer Organe, die die Durchblutung wiederspiegelt.

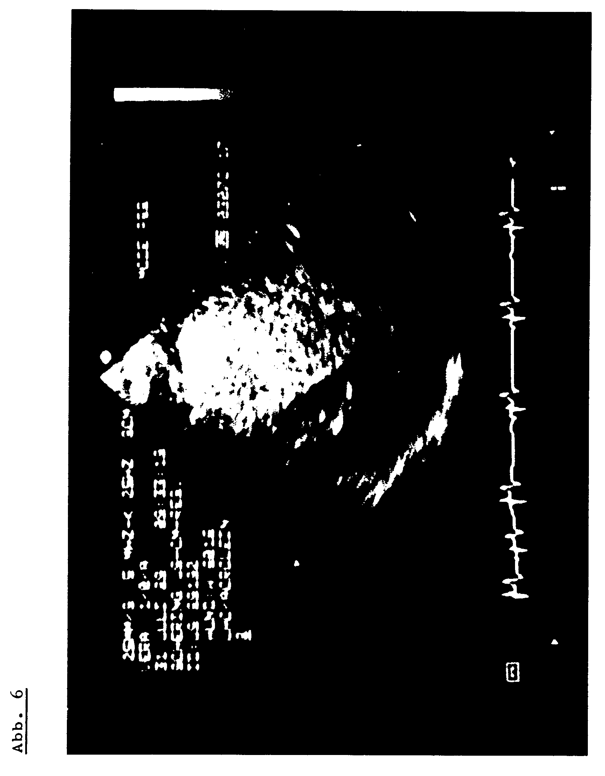

Abbildung 6 zeigt die Füllung des linken Ventikels mit Kontrastmittel.

Die Verwendung der erfindungsgemäßen Ultraschall-Kontrastmittel beschränkt sich nicht auf die Sichtbarmachung der Blutströme im Gefäßsystem, eine Kontrastierung von Körperhöhlen ist ebenso möglich. Bedingt durch die Durchblutungsdarstellung kann auch mit gutem Erfolg die Untersuchung anderer Organe mit diesen Kontrastmitteln erfolgen.

## Patentansprüche

1. Mikropartikel mit einem Partikeldurchmesser von 0,1 bis 40 µm bestehend aus
(a) einem bioabbaubaren Polymeren, erhältlich aus einem polymerisierbaren Aldehyd und
(b) einem Gas und/oder einer Flüssigkeit mit einem Siedepunkt unter 60 °C, dadurch gekennzeichnet, daß die Mikropartikel durch Trennung der gasenthaltenden von den nicht gasenthaltenden Partikeln erhältlich sind.

2. Mikropartikel nach Anspruch 1, dadurch gekennzeichnet, daß das bioabbaubare Polymer ein Copolymer ist, das einem polymerisierbaren Aldehyd und einem zur Copolymerisation fähigen Zusatz aufgebaut ist.

3. Mikropartikel nach Anspruch 1 oder 2, dadurch gekennzeichnet, daß das bioabbaubare Polymer über Crosslinker vernetzt ist.

4. Mikropartikel nach Anspruch 1 bis 3, dadurch gekennzeichnet, daß an das bioabbaubare Polymer ein Bio- oder Makromolekül gebunden ist.

5. Mikropartikel nach Anspruch 4, dadurch gekennzeichnet, daß das an das bioabbaubare Polymer gebundene Bio- oder Makromolekül über ein Kopplungsagenz gebunden ist.

6. Mikropartikel nach Anspruch 1 bis 5, dadurch gekennzeichnet, daß das bioabbaubare Polymer ein Tensid oder Tensidgemisch enthält.

7. Mikropartikel nach Anspruch 1 bis 6, dadurch gekennzeichnet, daß der polymerisierbare Aldehyd ausgewählt wird aus α-, β-ungesättigten Aldehyden, α-substituierten Acroleinderivaten oder Dialdehyden.

8. Mikropartikel nach Anspruch 7 worin der α-, β-ungesättigte Aldehyd Acrolein, Crotonaldehyd oder Propinaldehyd ist.

9. Mikropartikel nach Anspruch 7 worin das α-substituierte Acroleinderivat α-Methylacrolein, α-Chloroacrolein, α-Phenylacrolein, α-Ethylacrolein, α-Isopropylacrolein, α-n-Butylacrolein oder α-n-Propylacrolein ist.

10. Mikropartikel nach Anspruch 7 worin der Dialdehyd Glutaraldehyd, Succinaldehyd oder ein Derivat dieser ist.

11. Mikropartikel nach Anspruch 2 worin als zur Copolymerisation fähiger Zusatz α-substituiertes Acrolein, β-substituierte Acrolein, Ethylcyanacrylat, Methylcyanacrylat, Butylcyanacrylat, Hexylcyanacrylat, Methylmetacrylat, Vinylalkohol, Acrylsäure, Methacrylsäure, Acrylsäurechlorid, Methacrylsäurechlorid, Acrylnitril, Methacrylnitril, Acrylamide, substituiertes Acrylamid, Hydroxymethylmethacrylat, Mesityloxid, Dimethylaminoethylmethacrylat-2-vinylpyridin oder N-vinyl-2-Pyrrolidinon verwendet wird.

12. Mikropartikel nach Anspruch 1 bis 11 enthaltend als Gas und/oder Flüssigkeit mit einem Siedepunkt unter 60 °C Ammoniak, Luft, Helium, Neon, Argon, Xenon, Krypton, Schwefelhalogenide, Schwefelhexafluorid, Stickstoff, Kohlenstoffoxide, Sauerstoff, Wasserstoff, Methan, Ethan, Propan, Butan, Pentan, Neopentan, Isopentan, Cyclopentan, Ethylen, Propylen, Acetylen, 3,3-Dimethyl-1-Butin, 2,3-Pentadien, 2-Methyl-2-Buten, 2-Methyl-1,3-Butadien, 2-Butin, 2-Methyl-1-Buten, 3-Methyl-1-Buten, Methylenchlorid, 1,1-Dichlorethylen, Isopropylchlorid, Dibromdifluormethan, Brommethan, Dimethylether, Diethylether, fluorierte Ether, Dimethylaminoaceton, Propylenoxid, N-Ethylmethylamin, N-Ethyldimethylamin oder Furan.

13. Mikropartikel nach Anspruch 5 worin als Kopplungsagenz Hydroxylamin, Butylamin, Allylamin, Ethanolamin, Trishydroxymethylaminomethan, 3-Amino-1-propansulfonsäure, 5-Aminovaleriansäure, 8-Aminooctansäure, D-Glucosaminhydrochlorid, Aminogalactose, Aminosorbit, Aminomannit, Diethylaminoethylamin, Aniline, Sulfonilsäureamid, Cholin, N-Methylglucamin, Piperazin, 1,6-Hexandiamin, Harnstoff, Hydrazin, Glycin, Alanin, Lysin, Serin, Valin, Leucin, Peptide, Proteine, Albumin, Humanserumalbumin, Polylysin, Gelatine, Polyglycolamine, Aminopolyalkohole, Dextransulfate mit Aminogruppen, N-Aminopolyethylenglycol, N,N'-Diaminopolyethylenglycol, Antikörper, Immunoglobuline, 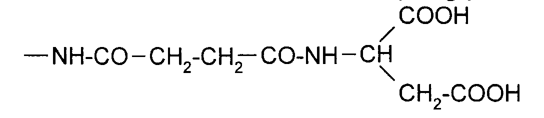 (PEG-Linker-Asparaginsäure), PEG-Linker-Gutaminsäure, PEG-Linker-DTPA, PEG-Linker-EDTA, Ethylenglycol, 1,3-Propandiol, Glycerin oder Pentaerythrit verwendet wird.

14. Mikropartikel nach Anspruch 4 enthaltend als Bio- oder Makromolekül monoklonale Antikörper.

15. Mikropartikel nach Anspruch 6 enthaltend als Tensid Polyethylenoxid, Pluronic® F 68, Pluronic® F 108, Pluronic® F 127, Polyethylenglycol, Poloxamin 908, Poloxamer 407, Natriumoleat, Polyoxyethylenstearat, Natriumdioctylsulfosuccinat, Polyglutaraldehydnatriumhydrogensulfit-Addukt, Polyacrolein-Natriumhydrogensulfid-Addukt oder Polyvinylsulfonsäure.

16. Pharmazeutische Mittel enthaltend Mikropartikel nach Anspruch 1 mit den in der Galenik üblichen Zusätzen.

17. Pharmazeutische Mittel nach Anspruch 16, dadurch gekennzeichnet, daß die Konzentration der Mikropartikel 1 µg bis 100 mg pro ml galenischer Formulierung beträgt.

18. Verwendung von Mikropartikeln nach Anspruch 1 in der Ultraschalldiagnostik.

19. Verfahren zur Herstellung von Mikropartikeln nach Anspruch 1, dadurch gekennzeichnet, daß man zu einer wäßrigen Lösung, die das gewünschte Gas und/oder Flüssigkeit mit einem Siedepunkt unter 60 °C enthält, unter Rühren bei einer Temperatur von -5 °C bis + 80 °C und einem pH-Wert von 7 bis 14 innerhalb von 1 Minute bis 24 Stunden den polymerisierbaren Aldehyd gibt, wobei die Konzentration des Aldehyds bezogen auf die Reaktionsmischung 0,1 bis 50% w/v beträgt und nach erfolgter Polymerisation gasenthaltende von nicht-gasenthaltenden Partikeln abtrennt und gewünschtenfalls anschließend reinigt.

20. Verfahren zur Herstellung von Mikropartikeln nach Anspruch 19, dadurch gekennzeichnet, daß man zu einer wäßrigen Lösung, die das gewünschte Gas und/oder Flüssigkeit mit einem Siedepunkt unter 60 °C enthält, unter Rühren bei einer Temperatur von -5 °C bis + 80 °C und einem pH-Wert von 7 bis 14 innerhalb von 1 Minute bis 24 Stunden den polymerisierbaren Aldehyd gibt, wobei die Konzentration des Aldehyds bezogen auf die Reaktionsmischung 0,1 bis 50% w/v beträgt und nach erfolgter Polymerisation gasenthaltende von nicht-gasenthaltenden Partikeln abtrennt, gewünschtenfalls reinigt und anschließend die so erhaltenen gashaltigen Partikel mit einer - bezogen auf die Aldehydmenge - bis zu äquimolaren Menge an Kopplungsagenz enthaltenden wäßrigen Lösung unter bis zu 3-tägigem Rühren bei einer Temperatur von 0 ° bis 60 °C bei einem pH-Wert von 3 bis 9 umsetzt und anschließend an Bio- oder Makromoleküle bindet.

21. Verfahren zur Herstellung von Mikropartikeln nach Anspruch 19, dadurch gekennzeichnet, daß man zu einer wäßrigen Lösung, die das gewünschte Gas und/oder Flüssigkeit mit einem Siedepunkt unter 60 °C enthält, unter Rühren bei einer Temperatur von -5 °C bis + 80 °C und einem pH-Wert von 7 bis 14 innerhalb von 1 Minute bis 24 Stunden den polymerisierbaren Aldehyd gibt, wobei die Konzentration des Aldehyds bezogen auf die Reaktionsmischung 0,1 bis 50% w/v beträgt und nach erfolgter Polymerisation gasenthaltende von nicht-gasenthaltenden Partikeln abtrennt, gewünschtenfalls reinigt und anschließend die so erhaltenen gashaltigen Partikel an Bio- oder Makromoleküle bindet.

22. Verfahren nach einem der Ansprüche 19 bis 21, dadurch gekennzeichnet, daß der polymerisierbare Aldehyd einen copolymerisierbaren Zusatz in einer Konzentration bis 20 % w/v enthält.

23. Verfahren nach einem der Ansprüche 19 bis 22, dadurch gekennzeichnet, daß der polymerisierbare Aldehyd einen Crosslinker in einer Konzentration bis 5 % w/v enthält.

24. Verfahren nach einem der Ansprüche 19 bis 23, dadurch gekennzeichnet, daß die Reaktionslösung ein Tensid in einer Konzentration 0,01 bis 10% w/v enthält.

25. Verfahren nach einem der Ansprüche 18 bis 24, dadurch gekennzeichnet, daß während der Polymerisation das jeweils gewünschte Gas eingeleitet wird.

## Claims

1. Microparticles having a particle diameter of from 0.1 to 40µm consisting of
(a) a biodegradable polymer obtainable from a polymerisable aldehyde and
(b) a gas and/or a liquid having a boiling point of less than 60°C,
characterised in that the microparticles are obtainable by separation of the gas-containing particles from the non-gas-containing particles.

2. Microparticles according to claim 1, characterised in that the biodegradable polymer is a copolymer that is synthesised from a polymerisable aldehyde and an additive capable of copolymerisation.

3. Microparticles according to claim 1 or 2, characterised in that the biodegradable polymer is cross-linked by means of cross-linkers.

4. Microparticles according to claims 1 to 3, characterised in that a bio- or macro-molecule is bound to the biodegradable polymer.

5. Microparticles according to claim 4, characterised in that the bio- or macro-molecule bound to the biodegradable polymer is bound by means of a coupling agent.

6. Microparticles according to claims 1 to 5, characterised in that the biodegradable polymer contains a surfactant or mixture of surfactants.

7. Microparticles according to claims 1 to 6, characterised in that the polymerisable aldehyde is selected from α,β-unsaturated aldehydes, α-substituted acrolein derivatives and dialdehydes.

8. Microparticles according to claim 7 wherein the α,β-unsaturated aldehyde is acrolein, crotonaldehyde or propynaldehyde.

9. Microparticles according to claim 7 wherein the α-substituted acrolein derivative is α-methylacrolein, α-chloroacrolein, α-phenylacrolein, α-ethylacrolein, α-isopropylacrolein, α-n-butylacrolein or α-n-propylacrolein.

10. Microparticles according to claim 7 wherein the dialdehyde is glutaraldehyde or succinaldehyde, or a derivative thereof.

11. Microparticles according to claim 2 wherein there is used as the additive capable of copolymerisation α-substituted acrolein, β-substituted acrolein, ethyl cyanoacrylate, methyl cyanoacrylate, butyl cyanoacrylate, hexyl cyanoacrylate, methyl methacrylate, vinyl alcohol, acrylic acid, methacrylic acid, acrylic acid chloride, methacrylic acid chloride, acrylonitrile, methacrylonitrile, acrylamides, substituted acrylamide, hydroxymethyl methacrylate, mesityl oxide, dimethylaminoethyl methacrylate-2-vinylpyridine or N-vinyl-2-pyrrolidinone.

12. Microparticles according to claims 1 to 11, containing as gas and/or liquid having a boiling point of less than 60°C ammonia, air, helium, neon, argon, xenon, krypton, sulphur halides, sulphur hexafluoride, nitrogen, carbon oxides, oxygen, hydrogen, methane, ethane, propane, butane, pentane, neopentane, isopentane, cyclopentane, ethylene, propylene, acetylene, 3,3-dimethyl-1-butyne, 2,3-pentadiene, 2-methyl-2-butene, 2-methyl-1,3-butadiene, 2-butyne, 2-methyl-1-butene, 3-methyl-1-butene, methylene chloride, 1,1-dichloroethylene, isopropyl chloride, dibromodifluoromethane, bromomethane, dimethyl ether, diethyl ether, fluorinated ethers, dimethylaminoacetone, propylene oxide, N-ethylmethylamine, N-ethyldimethylamine or furan.

13. Microparticles according to claim 5 wherein there is used as coupling agent hydroxylamine, butylamine, allylamine, ethanolamine, tris(hydroxymethyl)aminomethane, 3-amino-1-propanesulphonic acid, 5-aminovaleric acid, 8-aminooctanoic acid, D-glucosamine hydrochloride, aminogalactose, aminosorbitol, aminomannitol, diethylaminoethylamine, anilines, sulphanilic acid amide, choline, N-methylglucamine, piperazine, 1,6-hexanediamine, urea, hydrazine, glycine, alanine, lysine, serine, valine, leucine, peptides, proteins, albumin, human serum albumin, polylysine, gelatin, polyglycol amines, aminopolyalcohols, dextran sulphates having amino groups, N-aminopolyethylene glycol, N,N'-diaminopolyethylene glycol, antibodies, immunoglobulins, 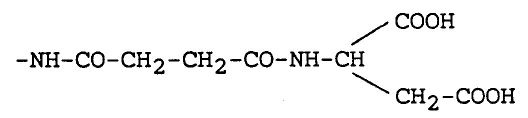 (PEG-linker-aspartic acid), PEG-linker-glutamic acid, PEG-linker-DTPA, PEG-linker-EDTA, ethylene glycol, 1,3-propanediol, glycerol or pentaerythritol.

14. Microparticles according to claim 4, containing monoclonal antibodies as bio- or macro-molecules.

15. Microparticles according to claim 6, containing as surfactant polyethylene oxide, Pluronic^{(R)} F 68, Pluronic^{(R)} F 108, Pluronic^{(R)} F 127, polyethylene glycol, poloxamin 908, poloxamer 407, sodium oleate, polyoxyethylene stearate, sodium dioctylsulphosuccinate, polyglutaraldehyde-sodium hydrogen sulphite adduct, polyacrolein-sodium hydrogen sulphide adduct or polyvinylsulphonic acid.

16. Pharmaceutical agents containing microparticles according to claim 1 together with the additives customary in galenical pharmacy.

17. Pharmaceutical agents according to claim 16, characterised in that the concentration of the microparticles is from 1 µg to 100 mg per ml of galenical formulation.

18. Use of microparticles according to claim 1 in ultrasound diagnostics.

19. Process for the preparation of microparticles according to claim 1, characterised in that the polymerisable aldehyde is added, with stirring at a temperature from -5° to +80°C and a pH value of from 7 to 14, in the course of from 1 minute to 24 hours to an aqueous solution that contains the desired gas and/or liquid having a boiling point of less than 60°C, the concentration of the aldehyde, based on the reaction mixture, being from 0.1 to 50% w/v, and after polymerisation has been carried out, the gas-containing particles are separated from the non-gas-containing particles and then, if desired, purified.

20. Process for the preparation of microparticles according to claim 19, characterised in that the polymerisable aldehyde is added, with stirring at a temperature from -5° to +80°C and a pH value of from 7 to 14, in the course of from 1 minute to 24 hours to an aqueous solution that contains the desired gas and/or liquid having a boiling point of less than 60°C, the concentration of the aldehyde, based on the reaction mixture, being from 0.1 to 50% w/v, and after polymerisation has been carried out, the gas-containing particles are separated from the non-gas-containing particles, purified if desired, and then the gas-containing particles so obtained are reacted with an up to equimolar amount - based on the amount of aldehyde - of aqueous solution containing coupling agent, with stirring for up to 3 days at a temperature of from 0° to 60°C and a pH value of from 3 to 9, and then bound to bio- or macro-molecules.

21. Process for the preparation of microparticles according to claim 19, characterised in that the polymerisable aldehyde is added, with stirring at a temperature from -5° to +80°C and a pH value of from 7 to 14, in the course of from 1 minute to 24 hours to an aqueous solution that contains the desired gas and/or liquid having a boiling point of less than 60°C, the concentration of the aldehyde, based on the reaction mixture, being from 0.1 to 50% w/v, and after polymerisation has been carried out, the gas-containing particles are separated from the non-gas-containing particles, purified if desired, and then the gas-containing particles so obtained are bound to bio- or macro-molecules.

22. Process according to any one of claims 19 to 21, characterised in that the polymerisable aldehyde contains a copolymerisable additive in a concentration of up to 20% w/v.

23. Process according to any one of claims 19 to 22, characterised in that the polymerisable aldehyde contains a cross-linker in a concentration of up to 5% w/v.

24. Process according to any one of claims 19 to 23, characterised in that the reaction solution contains a surfactant in a concentration of from 0.01 to 10% w/v.

25. Process according to any one of claims 18 to 24, characterised in that the desired gas is introduced in the course of the polymerisation.

## Revendications

1. Microparticules ayant un diamètre de particule de 0,1 à 40 µm composée
(a) d'un polymère biodégradable que l'on peut obtenir à partir d'un aldéhyde polymérisable et
(b) d'un gaz et/ou d'un liquide ayant un point d'ébullition inférieur à 60 °C,
caractérisées en ce que l'on peut obtenir les microparticules par séparation des particules contenant du gaz des particules ne contenant pas de gaz.

2. Microparticules selon la revendication 1, caractérisées en ce que le polymère biodégradable est un copolymère qui est constitué d'un aldéhyde polymérisable et d'un adjuvant copolymérisable.

3. Microparticules selon la revendication 1 ou 2, caractérisées en ce que le polymère biodégradable est réticulé à l'aide d'un agent réticulant.

4. Microparticules selon les revendications 1 à 3, caractérisées en ce que le polymère biodégradable est lié à une bio- ou macromolécule.

5. Microparticules selon la revendication 4, caractérisées en ce que la bio- ou macromolécule liée au polymère biodégradable est liée par l'intermédiaire d'un agent de couplage.

6. Microparticules selon les revendications 1 à 5, caractérisées en ce que le polymère biodégradable contient un agent de surface ou un mélange d'agents de surface.

7. Microparticules selon la revendication 6, caractérisées en ce que l'aldéhyde polymérisable est pris dans le groupe des aldéhydes α, β-insaturés, des dérivés d'acroléine α-substituée ou des dialdéhydes.

8. Microparticules selon la revendication 7, dans lesquelles l'aldéhyde insaturé en α, β est l'acroléine, le crotonaldéhyde ou le propionaldéhyde.

9. Microparticules selon la revendication 7, dans lesquelles le dérivé d'acroléine substitué en α est l'α-méthylacroléine, l'α-chloroacroléine, l'α-phénylacroléine, l'α-éthylacroléine, l'α-isopropylacroléine, l'α-n-butylacroléine ou l'α-n-propylacroléine.

10. Microparticules selon la revendication 7, dans lesquelles le dialdéhyde est le glutaraldéhyde, le succinaldéhyde ou un dérivé de ces aldéhydes.

11. Microparticules selon la revendication 2, dans lesquelles l'additif copolymérisable est l'acroléine substituée en α, l'acroléine substituée en β, le cyanoacrylate d'éthyle, le cyanoacrylate de méthyle, le cyanoacrylate de butyle, le cyanoacrylate d'hexyle, le méthacrylate de méthyle, l'alcool de vinyle, l'acide acrylique, l'acide méthacrylique, le chlorure d'acide acrylique, le chlorure d'acide méthacrylique, l'acrylonitrile, le méthacrylonitrile, les acrylamides, l'acrylamide substitué, le méthacrylate d'hydroxyméthyle, l'oxyde de mésityle, le diméthylaminoéthylméthacrylate de 2-vinylpyridine ou la N-vinyl-2-pyrrolidinone

12. Microparticules selon la revendication 1 à 11, contenant en tant que gaz et/ou en tant que liquide ayant un point d'ébullition inférieur à 60 °C, l'ammoniac, l'air, l'hélium, le néon, l'argon, le xénon, le krypton, des halogénures de soufre, l'hexafluorure de soufre, l'azote, des oxydes de carbone, l'oxygène, l'hydrogène, le méthane, l'éthane, le propane, le butane, le pentane, le néopentane, l'isopentane, le cyclopentane, l'éthylène, le propylène, l'acétylène, le 3,3-diméthyl-1-butyne, le 2,3-pentadiène, le 2-méthyl-2-butène, le 2-methyl-1,3-butadiène, le 2-butyne, le 2-méthyl-1-butène, le 3-méthyl-1-butène, le chlorure de méthylène, le 1,1-dichloroéthylène, le chlorure d'isopropyle, le dibromodifluorométhane, le bromométhane, l'éther diméthylique, l'éther diéthylique, des éthers fluorés, la diméthylaminoacétone, l'oxyde de propylène, la N-éthylméthylamine, la N-éthyldiméthylamine ou le furanne.

13. Microparticules selon la revendication 5, dans lesquelles l'agent de couplage est l'hydroxylamine, la butylamine, l'allylamine, l'éthanolamine, le trishydroxyméthylaminométhane, l'acide 3-amino-1-propanesulfonique, l'acide 5-aminovalérique, l'acide 8-amino-octanoïque, le chlorhydrate de D-glucosamine, l'aminogalactose, l'aminosorbitol, l'aminomannitol, la diéthylaminoéthylamine, l'aniline, le sulfonylamide, la choline, la N-méthylglucamine, la pipérazine, la 1,6-hexanediamine, l'urée, l'hydrazine, la glycine, l'alanine, la lysine, la sérine, la valine, la leucine, les peptides, les protéines, l'albumine, l'albumine sérique humaine, la polylysine, la gélatine, les polyglycolamines, les aminopolyalcools, le sulfate de dextrane avec des groupes amino, le N-aminopolyéthylèneglycol, le N,N'-diaminopolyéthylèneglycol, les anticorps, les immunoglobulines 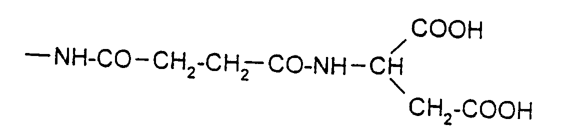 (PEG-lieur-acide asparagique), PEG-lieur-acide glutamique, PEG-lieur-DTPA, PEG-lieur-EDTA, l'éthylèneglycol, le 1,3-propanediol, la glycérine, le pentaérythritol

14. Microparticules selon la revendication 4 contenant en tant que bio- ou macromolécules des anticorps monoclonaux.

15. Microparticules selon la revendication 6 contenant en tant qu'agent de surface l'oxyde de polyéthylène, Pluronic® F 68, Pluronic® F 108, Pluronic® F 127, le polyéthylèneglycol, Poloxamin 908, Poloxamer 407, l'oléate de sodium, le stéarate de polyoxyéthylène, le dioctylsulfosuccinate de sodium, le produit d'addition de polyglutaraldéhyde et de bisulfite de sodium, le produit d'addition de polyacroléine et de bisulfite de sodium ou l'acide polyvinylsulfonique.

16. Agent pharmaceutique contenant des microparticules selon la revendication 1, avec des adjuvants usuels en galénique.

17. Agent pharmaceutique selon la revendication 16, caractérisé en ce que la concentration en microparticules est de 1 µg à 100 mg par ml de formulation galénique.

18. Utilisation des microparticules de la revendication 1 dans le diagnostic par ultrasons.

19. Procédé pour la préparation de microparticules selon la revendication 1, caractérisé en ce que l'on ajoute à une solution aqueuse qui contient le gaz et/ou le liquide ayant un point d'ébullition inférieur à 60 °C désiré, en agitant, à une température de -5 à +80 °C, et à une valeur de pH de 7 à 14, en l'espace d'une minute à 24 heures, l'aldéhyde polymérisable, la concentration en aldéhyde par rapport au mélange réactionnel étant de 0,1 à 50 % en poids/volume, et après polymérisation effectuée on sépare les particules contenant le gaz des particules ne contenant pas le gaz, et éventuellement on les purifie ensuite.

20. Procédé pour la préparation de microparticules selon la revendication 19, caractérisé en ce que l'on ajoute à une solution aqueuse qui contient le gaz et/ou le liquide ayant un point d'ébullition inférieur à 60 °C désiré, en agitant, à une température de -5 à +80 °C, et à une valeur de pH de 7 à 14, en l'espace d'une minute à 24 heures, l'aldéhyde polymérisable, la concentration en aldéhyde par rapport au mélange réactionnel étant de 0,1 à 50 % en poids/volume et après polymérisation effectuée, on sépare les particules contenant le gaz des particules ne contenant pas le gaz, éventuellement on les purifie et ensuite, on fait réagir les particules contenant du gaz ainsi obtenues avec une - par rapport à la quantité d'aldéhyde - quantité allant jusqu'à équimolaire en agent de couplage contenant la solution aqueuse en agitant jusqu'à 3 jours, à une température de 0 à 60 °C, à une valeur de pH de 3 à 9, et ensuite on lie à la bio- ou macromolécule.

21. Procédé pour la préparation de microparticules selon la revendication 19, caractérisé en ce que l'on ajoute à une solution aqueuse qui contient le gaz et/ou le liquide ayant un point d'ébullition inférieur à 60 °C désiré, en agitant, à une température de -5 à +80 °C, et à une valeur de pH de 7 à 14, en l'espace d'une minute à 24 heures, l'aldéhyde polymérisable, la concentration en aldéhyde par rapport au mélange réactionnel étant de 0,1 à 50 % en poids/volume et après polymérisation effectuée on sépare les particules contenant le gaz des particules ne contenant pas le gaz en purifiant éventuellement et ensuite en liant les particules contenant du gaz ainsi obtenues à la bio- ou macromolécule.

22. Procédé selon l'une des revendications 19 à 21, caractérisé en ce que l'aldéhyde polymérisable contient un additif copolymérisable en une concentration allant jusqu'à 20 % en poids/volume.

23. Procédé selon l'une des revendications 19 à 22, caractérisé en ce que l'aldéhyde polymérisable contient un agent réticulant en une concentration allant jusqu'à 5 % en poids/volume.

24. Procédé selon l'une des revendications 19 à 23, caractérisé en ce que la solution réactionnelle contient un agent de surface en une concentration de 0,01 à 10 % en poids/volume.

25. Procédé selon l'une des revendications 18 à 24, caractérisé en ce qu'au cours de la polymérisation on introduit le gaz respectif désiré.
